# EUROPEAN PATENT APPLICATION

(11) **EP 2 840 143 A1**
(43) Date of publication of application: **25.02.2015**
(21) Application number: 13181022.8
(22) Date of filing: 20.08.2013
(51) Int. Cl.: C12Q 1/00

(54) **A method for making a dry sensor element for an enzymatic determination of an analyte in a body fluid and a dry sensor element**

(71) Applicant: Roche Diagnostics GmbH, 68305 Mannheim (DE); F. Hoffmann-La Roche AG, 4070 Basel (CH)
(72) Inventor: Fürst, Otto, 68519 Viernheim (DE)
(74) Representative: Bittner, Thomas L.

(57) **Abstract**

The application relates to a method for making a dry sensor element for an enzymatic determination of an analyte in a body fluid, comprising: providing a substrate, producing a working electrode on the substrate, producing a counter electrode on the substrate, and producing electric connectors on the substrate connected to the working electrode and the counter electrode, the step of producing the working electrode further comprising depositing an active enzymatic electrode layer containing an enzyme and a catalyzer material for catalyzing an enzymatic determination of an analyte of a body fluid, the catalyzer material comprising at least one of metal nano-particles and metal oxide nano-particles. Further, a dry sensor element for an enzymatic determination of an analyte in a body fluid is disclosed.

## Description

The invention refers to a method for making a dry sensor element for an enzymatic determination of an analyte in a body fluid and a dry sensor element.

### Background

Electrochemical tests which may also be referred to as biosensors are used for the qualitative and / or quantitative analysis of ingredients in biological liquids such as, for example, blood, plasma, ISF or urine. One of the most important analyte is glucose. Examples of other analytes are lactate, PTT, pH, urea, lipids, ethanol, cholesterol and others. Examples of the implementation of electrochemical glucose tests are disclosed in US 5,413,690, US 5,762,770, US 5,798,031, US 5,997,817, US 2009 / 0020502 and WO 2009 / 056299.

Along with so-called point measurements in which a sample of a body fluid is specifically taken from a user and investigated for the analyte concentration, continuous measurements are increasingly becoming established. Continuous glucose measurement in the interstitium, also referred to as continuous monitoring or CM for short, has in the more recent past increasingly attracted attention and recognition as an important method for management, for monitoring and for control, for example, of a diabetes status. Directly implanted electrochemical sensors are usually now used, which are frequently also designated as needle type sensors (NTS). In this case the active sensor region is brought directly to the measurement site, usually located in the interstitial tissue, and for example, using an enzyme, for example glucose oxidase, glucose is converted into electrical current which is related to the glucose concentration and can be used as a measured variable. Examples of such transcutaneous measurement systems are described in US 6,360,888 B1, US 2008 / 0242962 Al and in WO 2007 / 071562 A1.

Implantable ectrochemical sensors usually have at least two or three electrodes. These are a substance-specific working electrode, a counter electrode and a reference electrode. The counter electrode can serve as a combi-electrode and also as reference. The substance-specific working electrode may be produced by applying an electrically conductive paste containing an enzyme, a mediator and a polymeric binder, to an electrical conductor path. For example, carbon pastes with manganese dioxide (MnO₂) and glucose oxidase (GOD) are commonly used. Examples of this are given in WO 2010 / 130833. Application is accomplished, for example, by means of a flat nozzle coating, by dispensing or by a printing process such as screen printing or ink jet (cf. Setti, et. al., Sensors and Actuators B 126 (2007) 252-257). A disadvantage of the coating of working electrodes by means of flat nozzle coating is the high material consumption of enzyme-containing and therefore expensive coating mass. The process does not allow exclusively the working electrode to be coated. On the contrary, the entire width of a test strip or sensor is coated and possibly also the intermediate space between two sensors.

The working electrode may be produced by a printing process, e.g. screen or porous printing. The material base of the working electrode may comprise a screen printing paste which must be electrically conductive (carbon pastes) and to which detection-specific components are added. These may be MnO₂, enzyme and other conductive carbon modifications. Conventional screen printing (carbon) pastes are optimized in relation to the composition of their components so that they meet the high requirements of the screen printing technique. In order to obtain the screen printing material for a functional working electrode from these pastes, further components are added to the screen printing paste. Such components are enzyme (GOD) (e.g. about 0,5 to 5 weight %), water (e.g. about 2 to 8 weight %), MnO₂ (e.g. about 12 to 20 weight %), and dispersing agent (e.g. about 5 to 15 weight %). It leads to substantial change of the nature of the original paste.

As an alternative to enzyme pastes, working electrodes can also be produced by electropolymerization of suitable monomers in the presence of enzymes. There is extensive literature on polypyrrole-based enzyme sensors. For example, the manufacture of enzyme electrodes based on polypyrrole was described (cf. Schuhmann in Methods in Biotechnology, Volume 6, Enzyme and Microbial Biosensors Techniques and Protocols, edited by A Mulchandani and K.R. Rogers, Humana Press Inc., Totaw NJ, page 143-156). The use of polyaniline as a base material for sensors is also frequently reported (cf. Arslan, et al., Sensors 2011, 11(8), 8152-8163).

The use of PEDOT:PSS ((poly(3,4ethylenedioxythiophene) : poly(styrenesulfonate)) for the production of glucose-sensitive working electrodes is described, for example, by Setti et al. in Biosensors and Bioelectronics, Volume 20, Issue 10, 2005, pages 2019-2026. Amperometric sensors based on electropolymerized PEDOT on platinum substrate are described by Nien et al., Electroanalysis (2006) 18, Issue 13-14, 1408-1415. A similar method is found in Fabiano et al., Mat. Sci. Eng. C-Bio. p. 2002, 21, 61. Further literature is listed in the review by N. Rozlosnik, Anal Bioanal Chem (2009) 395:3 p. 637. The use of water-containing preparations of monomeric EDOT with tenside and lithium perchlorate (LiClO₄) and its electropolymerization to dense closed PEDOT layers is described in WO 2000 / 063273. Good electrochemical stability of PEDOT:PSS compared with polypyrrole has been described by Yamato et al. in J. Electroanal Chem. 397 (1995) 163-170.

### Summary

It is an object of the invention to provide a method for making a dry sensor element for an enzymatic determination of an analyte in a body fluid and a dry sensor element which provide for efficient enzymatic determination of the analyte.

According to one aspect, a method for making a dry sensor element for an enzymatic determination of an analyte in a body fluid is provided. In the method a substrate is provided. On the substrate a working electrode and a counter electrode are produced. In addition, an electric connector is produced on the substrate which is connected to the working electrode and the counter electrode. In the step of producing the working electrode an active enzymatic electrode layer is deposited, the layer containing an enzyme and a catalyzer material for catalyzing an enzymatic determination of an analyte of a body fluid. The catalyzer material is containing at least one of metal nano-particles and metal oxide nano-particles.

According to another aspect, a dry sensor element for an enzymatic determination of an analyte in a body fluid is provided. The sensor element comprises a substrate, a working electrode on the substrate, a counter electrode on the substrate, and an electric connector on the substrate connected to the working electrode and the counter electrode. The working electrode is having an active enzymatic electrode layer containing an enzyme and a catalyzer material for catalyzing an enzymatic determination of an analyte of a body fluid, the catalyzer material containing at least one of metal nano-particles and metal oxide nano-particles.

A plurality of working electrodes may be provided. Also, the dry sensor element may be provided with a plurality of counter electrodes. The electric connectors will be connected to the one or more working electrodes and the one or more counter electrodes.

The dry sensor element may be provided as an implantable sensor element.

The method may further comprise depositing the active enzymatic electrode layer containing laser ablated nano-particles. Laser ablation of nano-particles at solids (blocks of metals or metal alloys) covered by a dispersing agent may carried out. Water may be used as the dispersing agent. A dispersion of nano-particles is prepared which can be used as a starting composition for further processing, e.g. for preparing a paste for printing the active enzymatic electrode layer. Metal oxides and dispersed powders in suitable dispersing agents can be (further) comminuted to nano-particles by the method. With respect to the nano-particles, several variants are feasible with regard to manufacture and processing. In one embodiment, Mn0₂ may be used. This substance may be dispersed in water and comminuted by laser. Furthermore suitable (macro-crystalline) manganese dioxide could be taken as the starting material and nano-MnO₂ particles may be produced from this (similar to the manufacture of nano Pt). The starting material would then, for example, be rhombic-crystallized pyrolusite (soft manganese ore) or tetragonally crystallized polianite.

The method may further comprise depositing the active enzymatic electrode layer by using a deposition process selected from the following group of deposition processes: electropolymerization, porous printing, and inkjet printing. As outlined above, the different technologies are known as such in various embodiments. If the method of screen or porous printing is used a paste containing the enzyme and the catalyzer material may be deposited for producing the active enzymatic electrode layer. If electro-polymerization is used for depositing the active enzymatic electrode layer, an electrolyte composition containing EDOT, PSS, and the catalyst material for electro-polymerization of PEDOT:PSS may be used, wherein the composition is provided in one of an emulsion, a solution, and a dispersion. The electrolyte composition may further contain polystyrene sulfonic acid or, one of its monovalent salts, as sodium or lithium or potassium, or ammonium salt. In one embodiment, an electropolymerization of EDOT to PEDOT:PSS may be done from an emulsion comprising non-ionic surfactant only by adding polystyrene sulfonic acid or one of its sodium, lithium, potassium or ammonium salts in water without adding conductivity enhancing salts.

The method may further comprise depositing the active enzymatic electrode layer containing nano-particles of at least one of the following materials: MnO₂, Pt, and Pd. The diameter of the nano-particles may be in average 2 nm to 60 nm, preferably 10 to 20 nm, further preferred about 15 nm.

The method may further comprise depositing the active enzymatic electrode layer containing at least one of GOD (glucose oxidase) and LOC (lactate oxidase).

The method may further comprise depositing the active enzymatic electrode layer containing carrier particles, the nano-particles being adsorb on a surface of the carrier particles.

The carrier particles may comprise carbon particles.

The method may further comprise depositing the active enzymatic electrode layer from a dispersion containing the nano-particles.

The method may comprise applying at least one of a membrane cover and a biocompatible cover. The covering may cover the working electrode at least partially. In addition, other parts of the surface of the electrode element may be covered. By the covering an implantable sensor element may be prepared. A biocompatibility layer may be applied in the form of a membrane which covers the sensor element at least partially in the region which comes in contact with the body of a living being. The application of a biocompatibility layer may be accomplished by dipping or spray coating. The application of a membrane may be accomplished, for example, from a solution consisting of a readily volatile solvent, optionally a less volatile less good solvent and one or more membrane-forming polymers. Dipping, printing, here in particular screen printing, or spray coating may serve as possible application methods.

For use of the dry sensor in living beings sterility may be provided for at least the part of the sensor element that comes in contact with the body of a living being. At least the part of the sensor element that comes in contact with the body of a living being may be packaged into an aseptic package and subjected to a sterilising radiation, for example electron beam, beta radiation and / or gamma radiation. Depending on potential precontamination a dose sufficient to ensure sterility is chosen. 25 kGray normally is considered an effective radiation dose.

The method may further comprise producing a reference electrode on the substrate.

With respect to the dry sensor element, variations or embodiments of the method outlined may apply accordingly.

According to still another aspect, an article comprising a dry sensor element is provided, wherein the sensor element is sterile tight packaged and radiation sterilized. In case of use the sterile packaging may be opened at least in part, for example, for establishing an electrical connection to electric connectors. The sterile packaging may be kept at least for the part of the sensor element which is to come into contact with the body of the living being.

### Description of embodiments

Following, embodiments will be described in further detail, by way of example. In the figures, show:
Fig. 1 a schematic representation of a base pattern for producing a dry sensor element, and
Fig. 2 a graphic representation of experimental results of a sensor signal: current (nA) in dependence on glucose concentration (mmol/1).

Laser ablation of nanoparticles may be carried out at solids (blocks of metals or metal alloys) covered by a dispersing agent which may be water. Following enquiries at the manufacturer however, metal oxides and dispersed powders in suitable dispersing agents can be (further) comminuted to nanoparticles by the method (for examples, see below).

With respect to the nano-particles, several variants are feasible with regard to manufacture and processing.

In one embodiment, it is started from MnO₂. To this end, this substance may be dispersed in water and comminuted by laser. Furthermore suitable (macro-crystalline) manganese dioxide could be taken as the starting material and nano-MnO₂ particles could be produced from this (similar to the manufacture of nano-particle of Pt). The starting material may then, for example, be rhombic-crystallized pyrolusite (soft manganese ore) or tetragonally crystallized polianite. During laser treatment locally high temperatures are produced and from 600°C (under classical conditions) Mn₃O₄ is formed from MnO₂ with release of O₂. Whether this takes place during the production of nano-particles and what the catalytic efficiency of Mn₃O₄ is would need to be checked and possibly the Mn₃O₄ could then be oxidized to MnO₂.

In another embodiment, it was found feasible to generate metallic Mn nano-particles which are introduced into the formulation as such and as mentioned above, are oxidized at a later time on their surface to give MnO₂. Here the particles can therefore be provided with an MnO₂ surface in their original aqueous dispersion before mixing into the paste, e.g. again by means of an auto-oxidation of the Mn by oxygen (air) in corresponding pH-adjusted medium or by means of suitable oxidation, e.g. by ozone / HCl + H₂O₂ or after processing again by mild oxidation, e.g. by H₂O₂, this generated by addition of glucose to GOD or by another form of oxidation to MnO₂.

In the event that Mn nanoparticles are too reactive, it was found also be feasible to take Re, homologous to Mn, or a Mn-Re alloy or another Mn alloy.

In still another embodiment, it was started with material magnetic manganese-containing oxide or mixed oxide.

In a further embodiment, magnetic spinels may be used as a starting substance. Redox-capable nanoparticles are produced from such compact mixed oxide structures which already contain Mn oxidized, e.g. of Mn₃O₄ or, for example of FeMnO mixed oxides, i.e. of Fe₂MnO₄, i.e. generally of spinels, similar to Fe₃O₄(2 Fe III + 1 Fe II + O₃). These particles themselves can already be catalytically active or the Mn(II+) present in them could be oxidized by means of MnO₄- to MnO₂ at the surface. Spinels, and therefore also these particles, are magnetic and therefore may, for example, more easily be concentrated prior to a further use.

For preparing a composition, the nano-particles produced by the different methods outlined above may be processed as follows (possibly after conversion into catalytically active species).

The dispersion in which they were generated may be used. That is, the dispersion as such is combined with the other components of the paste. Ideally the dispersing agent would then be identical to a liquid used in any case in the paste.

In another embodiment, the dispersion in which the nano-particles were generated is used with the addition of enzyme. That is, the dispersion is mixed with enzyme, e.g. GOD or LOT, and then combined with the other components of the paste.

In still another embodiment, the dispersion of the nano-particles is bound or can be bound to other particles which are part of the screen printing paste and these are then combined with the other components of the paste. For example, a specific quantity of conductive C-based material could be added to the nano-particle dispersion. This C-based material could in any case be an additional component of the paste, in order, for example to improve its conductivity / porosity etc. The nanoparticles may then adsorb into the C-particles with the advantages that they can now be separated "in a nano-particle manner" and can be applied into the paste and that they have an improved contact with the conductive elements of the paste. The enzyme could be added as a further component.

In a further embodiment, starting from the dispersion mentioned above a magnetic separation of magnetic nano-particles and therefore the concentration thereof may be done to form a mixture containing nano-particles. Following, this mixture is incorporated into the printing paste is commercially available as such.

Following, further embodiments are described. Enzyme electrodes, e.g. for an implantable dry sensor element, e.g. for glucose or lactate, are fabricated. The enzyme electrode providing a working electrode can be fabricated in substantially one working step by electropolymerization of EDOT to PEDOT:PSS in the presence of a catalyst and GOD in a controlled manner.

To this end, a micro-emulsion or micro-dispersion or solution of EDOT, PSS, respective monovalent salts thereof, nanoscale platinum and enzyme in an aqueous detergent solution may be used as electrolyte. Compared with the prior art it was found that an electropolymerization of EDOT to PEDOT:PSS was possible from an emulsion comprising non-ionic surfactant without adding conducting salts or oxidizing agents such as LiClO₄ only by adding polystyrene sulfonic acid or one of its sodium, lithium, potassium or ammonium salts in water to a layer having a large surface. The emulsion or dispersion may contain EDOT, non-ionic surfactant, PSS or PSS salt, water and optionally other components. As used here, such composition may be referred to as electrolyte. The description of the composition including the method of making it may be referred to as formulation.

In one embodiment of the formulation, a mass ratio of EDOT to surfactant to water of 10:43:20 was found to be relatively robust to additional fractions of water in the electrolyte and gives stable single-phase emulsions. Furthermore, by adding an aqueous dispersion of nano-scale platinum it is possible to incorporate a catalytic activity, e.g. to H₂O₂ or azide ions into the electropolymerized layer.

By adding GOD to the electrolyte an enzymatically active layer can be electropolymerized onto the working electrode of a sensor base pattern in one step. In this embodiment, the use of soluble mediators such as ferrocene is not required.

From aqueous solutions having pH 7.4 to pH 3.2, GOD can be adsorbed onto an electropolymerized nanoscale-platinum containing PEDOT:PSS layer on a working electrode so that even without application of a membrane, a sensor is obtained which showed constantly high sensitivity to glucose over several hours.

In one embodiment, it is surprisingly observed that the signals of sensor elements in the presence of H₂O₂ or glucose increase when the fraction of PSS-Na is reduced in electrolytes from which the working electrode is electropolymerized.

By adding glucose to the electrolyte, the signals of sensors thus fabricated increase.

So-called base patterns may be used for the electropolymerization, which may include the substrate, the conductor paths, the contacts, the electrode surfaces and the insulating layer, where the electrode surfaces are not yet provided with functionality. In the base pattern a plastic film, preferably made of PET or polyimide may be used as substrate for the electrode material and as base material for an optionally implantable sensor. The substrate can be reinforced by another material such as plastic or metal, in the case of metal, preferably stainless steel.

Contacts, conductor paths and electrode base surfaces in the base patterns may consist of metal, preferably Au, Pt, Pd or alloys thereof. Surprisingly, PEDOT:PSS was also found suitable. In the same way, it is possible to use contacts, conductor paths and electrode base surfaces of carbon pastes. Au may be used as a sputtered-on layer having a thickness of 40 to 100 nm or as a layer having a thickness of about 0.2 to about 1 µm applied galvanically to Cu, Ag or Ni. Pd and Pt may be used as sputtered layers having a thickness, for example, of about 50 nm. Carbon and PEDOT:PSS pastes may be applied by squeegee and structured or applied to a plastic substrate structured in screen printing.

A plastic layer, preferably an insulating varnish layer, e.g. a UV-curable insulating varnish layer, is used to insulate conductor paths on a base pattern for an optionally implantable sensor element. The insulating varnish layer can be applied structured in a printing process. Alternatively, it can be applied over the entire surface and structured by photolithographic methods so that the electrode surfaces and the contact regions are not covered. The insulating layer can also comprise an adhesive layer and fixed by adhesion.

Several base patterns for sensors can be combined in one unit and separated before or after manufacturing the sensors. The separation is accomplished by cutting, stamping or laser cutting.

The enzyme sensor element comprises at least one working electrode and a counter electrode. Preferably a reference electrode is also included. The functionality of the reference electrode is determined by Ag/AgCl. To this end, a corresponding Ag/AgCl paste is applied for example by printing by screen printing or metallic silver is applied and reacted with chloride. Other reference systems can be achieved, but may be unsuitable for an application in an implantable sensor. The functionality of the counter electrode is obtained, for example, by the electrode material used or by printed on carbon paste. The functionality of the working electrode may be achieved by the electropolymerization or by the electropolymerization and a consecutive adsorption of enzyme.

When using base patterns, the counter electrode may be also used as counter electrode for the electropolymerization. An experimental or production arrangement is thereby simplified. The areas of the electrodes and their spacings are fixed by the base pattern.

In the case of galvanostatic deposition of the active coating on the working electrode an increase in the signal level is found with increasing charge = deposition time at constant current.

With increasing current density from 2.5 A/m² to 80 A/m², an increase in the signal level from correspondingly produced sensors is observed with H₂O₂ and with glucose as analytes. At current intensities above 80 to 100 A/m² in the formulations used, the electrode potential increases significantly above 0.8 V and then the process of water oxidation with the formation of oxygen begins parallel to the electropolymerization. This results in a further structured surface of the deposited PEDOT and also in reduced adhesion. In addition, the linear relationship between charge and deposited mass of PEDOT:PSS is thereby lost and with this the possibility for exact monitoring of the electropolymerization process. It was further observed that with increasing current density the deposition product is more severely fissured and therefore has a larger external surface.

Another advantage of the PEDOT:PSS deposited at higher current density is the higher stability to dimensional variations during washing and drying. During washing after the electropolymerization, the PSS fraction absorbs water and expands, during drying the water is released again and the PEDOT:PSS shrinks. With smooth dense deposits this results in an increased tendency to flaking. This is observed particularly during the deposition of PEDOT:PSS on base patterns with sputtered gold on the working electrodes. Sputtered gold layers are significantly smoother than the galvanically deposited gold layers used as an alternative.

For the production of larger numbers of items, a plurality of base patterns may be used in one panel. The working electrodes of all the base patterns may be connected individually or jointly parallel as anodes to a regulated current supply. The working electrodes are individually wetted with electrolyte via respectively one capillary. The capillary is preferably designed so that it rests on the insulating layer surrounding the working electrode and brings about an extensive sealing. Located inside the capillary at a defined distance of 300 to 2000 µm from the end facing the base pattern is an electrical conductor serving as a cathode, preferably a gold wire or a gold layer. This cathode is connected to a regulated voltage supply. The regulated voltage supply supplies the plurality of base patterns with individually regulated constant current having an adjustable intensity and thereby brings about the electropolymerization on the working electrode of the sensors to be fabricated. By means of a piston or peristaltic pump a defined volume of the electrolyte is deposited on the base pattern during each raising of the capillary from a base pattern. As a result, during placement of the capillary on a next base pattern fresh electrolyte is always available at the tip. The working electrodes are washed after the electropolymerization by rinsing with distilled water by means of a water jet or by guiding the panel through one or more rinsing baths.

When drying sensors with electropolymerized PEDOT:PSS as working electrode in air or in vacuum, for one embodiment a severe decrease in sensitivity is observed. By adding 0.01% to 4.5% organic solvents such as THF, 1-propanol, DEGMBE, glycerine or 1-methoxy-2-propyl acetate to the last washing solution, it is possible to fabricate dry sensors whose activity to H₂O₂ or glucose is almost exactly as high as for non-dried sensors. By additionally adding 0.01% to 0.15% sugar or sugar alcohols such as trehalose (preferably), sorbitol, maltitol, galactose, lactitol or stabilizers such as di-inositol phosphate to the last washing solution, the sensitivity to glucose after drying in vacuum at 50°C for 10 min and storing at room temperature in air for four weeks can be maintained at more than 70% in relation to the sensitivity of sensors which have never been dried but otherwise fabricated in the same way.

The use of the sensors as implantable or partially implantable sensors for the continuous measurement (CM) of glucose or other analytes in the tissue requires adaptation of the removal transport of analyte and its reaction partner oxygen to the working electrodes and the removal of reaction products from the working electrodes through a suitable membrane. This membrane may further provide for preventing the out-diffusion of enzymes from the working electrodes into the tissue since the enzyme is not compatible for the tissue and without a membrane the biocompatibility of such a sensor possibly would not be ensured.

An improvement in the biocompatibility of such a sensor can be achieved by final application of a biocompatibility layer in the form of a membrane which covers the sensor at least partially in the region which comes in contact with the body of a living being. The application of a biocompatibility layer may be accomplished by dipping or spray coating.

The application of a membrane may be accomplished, for example, from a solution consisting of a readily volatile solvent, optionally a less volatile less good solvent and one or more membrane-forming polymers. Dipping, printing, here in particular screen printing, or spray coating serve as possible application methods.

Following, examples are described.

In a first example, an enzyme-containing electrolyte is prepared. An electrolyte containing GOD (glucose oxidase) is prepared. The following components are used:
- 64 parts by weight of Lutensol NO 110, Article No. 50070496, BASF SE, Ludwigshafen, Germany
- 5 parts by weight of 3% aqueous solution of poly-(sodium-4-styrene sulfonate) 30%, Article No. 527483, Sigma-Aldrich Chemie GmbH, Munich, Germany
- 15 parts by weight of ethylene 3,4-dioxythiophene, Clevios M V2, Heraeus Precious Metals GmbH & Co, Hanau, Germany;
- 50 parts by weight of platinum nanoparticle suspension in water (500 mg/l), catalogue No. 78-1410, Strem Chemicals GmbH, Kehl, Germany;
- 2 parts by weight of glucose oxidase rec. Lyo RGI (>200 U/mg Lyo) Mat No.: 11939998 106, Roche Diagnostics GmbH, Mannheim, Germany;
- 10 parts by weight citrate buffer 10 mM, pH = 3.5.

The components are successively placed in a suitable vessel made of PP at room temperature, mixed intensively after each addition and used for electropolymerization.

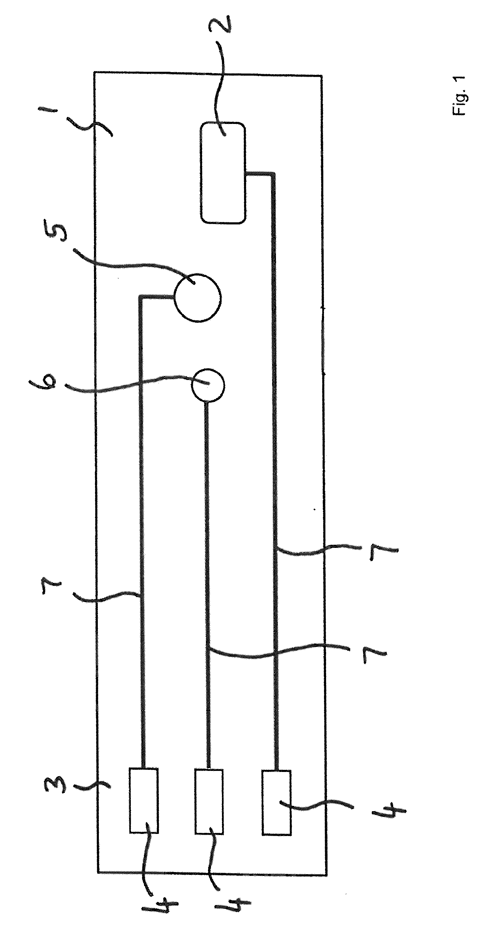

For preparation of a sensor element, a flexible sensor base pattern with polyimide as substrate and galvanically gold-plated conductor paths and base patterns is provided. Such flexible pattern is available, for example, from Dyconex AG, Bassersdorf, Switzerland. Fig. 1 shows such a substrate 1. A distal base structure 2 furthest away from a plug region 3 provided with connector pads 4 is used as counter electrode, a middle base structure 5 is used for producing a working electrode, and a proximal base structure 6 located nearest to the plug region 3 is used as reference electrode. The connector pads 4 are connected to the plurality of base structures by conductor paths 7.

An Ag/AgCl paste is metered onto the reference electrode surface with a metering device. After drying, an Ag/AgCl reference electrode is present.

For electropolymerization, the sensor basic pattern is electrically connected in the plug region to a power source Source Meter 2400, Keithley Instruments Inc. Solon, USA, where the working electrode surface is switched as anode and the counter electrode surface is switched as cathode. Contact with the reference electrode is not absolutely essential but can be accomplished to monitor the electrode potentials with a suitable power source. The contacted sensor base pattern is dipped into the preparation described above at least so far that the reference electrode is in contact with the electrolyte. In the case of a galvanostatic deposition at a current density of 28.3 A/m² for 100 s a well-adhering layer of PEDOT:PSS with incorporated nanoscale platinum particles and GOD is formed. The sensor thus formed is washed with distilled water and is available for measurement of glucose in PBS buffer.

Following, a measurement is made using a self-built potentiostat. Commercially available potentiostats having a suitable sensitivity in the pico-ampere range such as for example Ref erence 600^{™} from Gamry Instruments are also suitable.

The sensor element prepared before is dipped at least until the reference electrode is completely covered in PBS buffer. The liquid is not stirred during the measurement. Stirring or circulating is possible but requires reproducible conditions and in individual cases gives somewhat different results than without liquid movement. A voltage of 350 mV or 500 mV is applied between working electrode and counter electrode and the current is measured. Typically in PBS buffer there is a high initial current which decreases within 600 to 1800 sec asymptotically to close to 0 nA. During a measurement before reaching the zero value the drifting zero line should be taken into account when determining the signal height.

Different H₂O₂ or glucose concentrations are prepared by supplying hydrogen peroxide or glucose solution in PBS buffer or by exchanging the vessel with the liquid to be investigated. With increasing analyte content in the PBS buffer, the current increases.

A current of 25.9 nA is observed with 6.5 mmol/glucose/l.

The sensor prepared may be dried in air at room temperature for 2 h, alternatively at 50°C for 30 min. During the measurement as outlined above a signal of 3.6 nA is observed.

The sensor prepared may be rinsed for 20 sec in an aqueous solution of 10 g/l 1-propanol and then dried as outlined above. During the measurement as explained above a signal of 21.2 nA is observed.

In a second example, an electrolyte without enzyme is prepared. The following preparation of an electrolyte is prepared:
- 64 parts by weight of Lutensol ON 110, Article No. 50070496, BASF SE, Ludwigshafen, Germany;
- 5 parts by weight of 3% aqueous solution of poly-(sodium-4-styrene sulfonate) 30%, Article No. 527483, Sigma-Aldrich Chemie GmbH, Munich, Germany;
- 15 parts by weight of ethylene dioxythiophene, Clevios M V2, Heraeus Precious Metals GmbH & Co, Hanau, Germany; and
- 50 parts by weight of platinum nanoparticle suspension in water (500 mg/l), catalogue No. 78-1410, Strem Chemicals GmbH, Kehl, Germany.

The components are successively placed in a suitable vessel made of PP at room temperature, mixed intensively after each addition and used for electropolymerization.

For electropolymerization, a sensor base pattern (cf. explanation given above) is connected to a power source as outlined above and dipped at least as far into the preparation that the reference electrode is in contact with the electrolyte. In the case of a galvanostatic deposition at a current density of 61 A/m² for 100 sec a well-adhering layer of PEDOT:PSS with incorporated nanoscale platinum particles is formed. The sensor is washed intensively with distilled water and stored moist.

An enzyme-containing solution is prepared, namely a GOD containing solution. Citrate buffer having a concentration of 20 mM with a pH of 3.5 is prepared from citric acid and sodium citrate according to established laboratory practice. 100 mg of lyophilized GOD glucose oxidase rec. Lyo RGI (>200 U/mg Lyo) Mat No.: 11939998 106, Roche Diagnostics GmbH, Mannheim, Germany was weighed into 1 aliquot of 1 ml of this citrate buffer, mixed intensively for 1 min with a vortex mixer and then stored for 30 min on a slow-running roll mixer (0.1 rpm). After storing for a further 30 min at rest the GOD solution is ready to use.

GOD contained in the solution prepared may be adsorbed on the electropolymerized working electrode prepared before. A sensor prepared by electropolymerization is briefly centrifuged to remove any adhering droplets and dipped in the preparation (GOD solution) at room temperature for 300 sec at least as far as the reference electrode. Excess GOD is removed by washing in distilled water for at least 2 x 10 s. The sensor is rinsed for 20 sec in an aqueous solution of 10 g/l 1-propanol and dried for 10 min at room temperature in air and for 30 min at 50°C in a drying cabinet. The sensor element is then stored in an Eppendorf vessel at room temperature until the measurement. During measurement with 1.625 mM glucose in PBS as outlined above, a signal of 95 nA is observed.

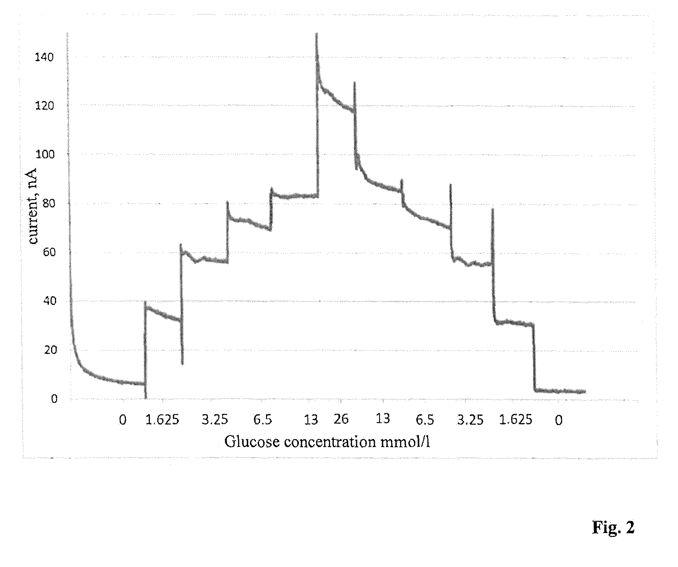

The sensor provided with GOD adsorbed is dipped in a 1.95 percent solution of Tecophilic TG-500 in THF/1-propanol (1+3) and extracted at a speed of 0.2 cm/s. After aerating for 10 minutes at room temperature in stationary air, the sensor is dried for 30 min at 50°C in a drying cabinet. During measurement with solutions of 0 to 26 mMol of glucose in PBS buffer, according to Fig. 2, a stepwise increase in the measurement signal from 6 nA to 120 nA is obtained.

The embodiments described may provide for one or more of the following advantages. A low material consumption compared with a squeegee coating. The mass of the active working electrode produced by electropolymerization can be controlled exactly by detecting the expended electrical charge (current x time). By using non-metallic conductor paths such as PEDOT:PSS or carbon, expensive noble metal as gold may be avoided. By using counter electrodes on a sensor base pattern, a simple fabrication structure is possible with the electro-polymerization. In an embodiment, dry sensors with improved sensitivity can be obtained by using organic solvents in the last washing solution.

## Claims

1. A method for making a dry sensor element for an enzymatic determination of an analyte in a body fluid, comprising:
- providing a substrate,
- producing a working electrode on the substrate,
- producing a counter electrode on the substrate, and
- producing electric connectors on the substrate connected to the working electrode and the counter electrode,
the step of producing the working electrode further comprising depositing an active enzymatic electrode layer containing an enzyme and a catalyzer material for catalyzing an enzymatic determination of an analyte of a body fluid, the catalyzer material comprising at least one of metal nano-particles and metal oxide nano-particles.

2. Method of claim 1, further comprising depositing the active enzymatic electrode layer containing laser ablated nano-particles.

3. Method of claim 1 or 2, further comprising depositing the active enzymatic electrode layer by using a deposition process selected from the following group of deposition processes: electropolymerization, porous printing, and inkjet printing.

4. Method of one of the preceding claims, further comprising depositing the active enzymatic electrode layer containing nano-particles of at least one of the following materials: MnO₂, Pt, and Pd.

5. Method of one of the preceding claims, further comprising depositing the active enzymatic electrode layer containing at least one of glucose oxidase and lactate oxidase.

6. Method of one of the preceding claims, further comprising depositing the active enzymatic electrode layer containing carrier particles, the nano-particles being adsorb on a surface of the carrier particles.

7. Method of claim 6, the carrier particles comprising carbon particles.

8. Method of one of the preceding claims, further comprising depositing the active enzymatic electrode layer from a dispersion containing the nano-particles.

9. Method of one of the preceding claims, further comprising applying at least one of a membrane cover and a biocompatible cover.

10. A dry sensor element for an enzymatic determination of an analyte in a body fluid, comprising:
- a substrate,
- a working electrode on the substrate,
- a counter electrode on the substrate, and
- electric connectors on the substrate connected to the working electrode and the counter electrode,
the working electrode comprising an active enzymatic electrode layer containing an enzyme and a catalyzer material for catalyzing an enzymatic determination of an analyte of a body fluid, the catalyzer material comprising at least one of metal nano-particles and metal oxide nano-particles.

11. The dry sensor element of claim 15, wherein at least the working electrode is provided with at least one of a membrane cover and a biocompatible cover.
